(19) Europäisches Patentamt
European Patent Office
Office européen des brevets

(11) Veröffentlichungsnummer: **0 001 279**
**B1**

(12) **EUROPÄISCHE PATENTSCHRIFT**

(45) Veröffentlichungstag der Patentschrift: **30.09.81**

(21) Anmeldenummer: **78100923.8**

(22) Anmeldetag: **18.09.78**

(51) Int. Cl.³: **C 07 D 401/12,**
C 07 D 403/12,
C 07 D 417/12,
A 61 K 31/415

(54) **Imidazolderivate deren Herstellung und diese enthaltende Präparate**

(30) Priorität: **19.09.77 LU 78140**
**28.07.78 CH 8149/78**

(43) Veröffentlichungstag der Anmeldung:
**04.04.79 Patentblatt 79/07**

(45) Bekanntmachung des Hinweises auf die Patenterteilung:
**30.09.81 Patentblatt 81/39**

(84) Benannte Vertragsstaaten:
**BE CH DE FR LU NL SE**

(56) Entgegenhaltungen:
**DE - A - 2 548 340**

(73) Patentinhaber: **F. HOFFMANN-LA ROCHE & CO. Aktiengesellschaft**
**CH-4002 Basel (CH)**

(72) Erfinder: **Krassó, Anna, Dr.**
**Rütimeyerstrasse 35**
**CH-4045 Basel (CH)**
Erfinder: **Krebs, Ernst-Peter, Dr.**
**Spitzackerstrasse 104**
**CH-4103 Bottmingen (DE)**

(74) Vertreter: **Lederer, Franz, Dr., et al**
**Patentanwälte Lederer**
**Meyer Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 001 279 B1

Courier Press, Leamington Spa, England.

# Imidazolderivate, deren Herstellung, und diese enthaltende Präparate

Die vorliegende Erfindung betrifft Imidazolderivate der allgemeinen Formel

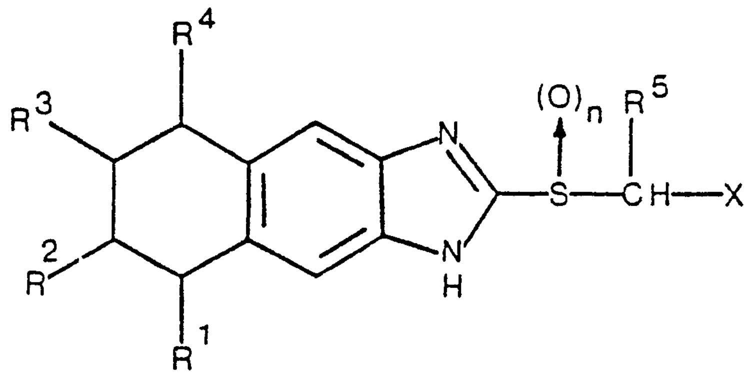

I

worin $R^1$, $R^2$, $R^3$ und $R^4$ je Wasserstoff oder $R^1$ zusammen mit $R^2$ und $R^3$ zusammen mit $R^4$ je eine zusätzliche Kohlenstoff-Kohlenstoff-Bindung, n die Zahl 0 oder 1, $R^5$ Wasserstoff oder niederes Alkyl und X einen gegebenenfalls durch niederes Alkyl monosubstituierten 2-Pyridylrest, einen 2-Imidazolylrest, einen 2-Imidazolinylrest, einen 2-Thiazolylrest, einen 2-Thiazolinylrest, oder einen gegebenenfalls durch niederes Alkyl monosubstituierten 4(5)-Imidazolylrest bedeuten, und deren Säureadditionssalze.

Der Ausdruck "niederes Alkyl" bezeichnet geradkettige oder verzweigte, gesättigte, aliphatische Kohlenwasserstoffreste mit bis zu 7, vorzugsweise bis zu 4 Kohlenstoffatomen, wie Methyl, Aethyl, n-Propyl, Isopropyl.

Unter den Verbindungen gemäss vorliegender Erfindung sind diejenigen bevorzugt, worin X einen gegebenenfalls durch niederes Alkyl monosubstituierten 2-Pyridylrest bedeutet. Besonders bevorzugt ist der Methyl-substituierte 2-Pyridylrest. Unter den Alkyl-substituierten 2-Pyridylresten sind diejenigen mit 5- oder 6-ständiger Methylgruppe besonders bevorzugt. $R^5$ ist vorzugsweise Wasserstoff. Weiterhin sind unter den Verbindungen gemäss vorliegender Erfindung diejenigen bevorzugt, worin $R^1$ zusammen mit $R^2$ und $R^3$ zusammen mit $R^4$ je eine zusätzliche Kohlenstoff-Kohlenstoff-Bindung bedeuten und diejenigen, in denen $n = 0$ ist.

Strukturähnliche, im Benzolring gegebenenfalls durch einwertige Reste mono- oder disubstituierte Benzimidazol-derivate sind in der Deutschen Offenlegungsschrift No. 2.548.340 und in der Britischen Patent Specification No. 1.234.058 beschrieben.

Die Erfindung betrifft weiterhin ein Verfahren zur Herstellung der Verbindungen I und pharmazeutische Präparate davon.

Ganz besonders bevorzugt sind das 2-[/(5-Methyl-2-pyridyl)methyl/thio]-1H-naphth[2,3-d]-imidazol und das 2-[(2-Pyridylmethyl)thio]-1H-naphth[2,3-d]imidazol.

Verbindungen der allgemeinen Formel I und deren Säureadditionssalze können erfindungsgemäss dadurch hergestellt werden, dass man

a) zur Herstellung von Verbindungen der Formel I, worin n die Zahl 0 bedeutet, eine Verbindung der allgemeinen Formel

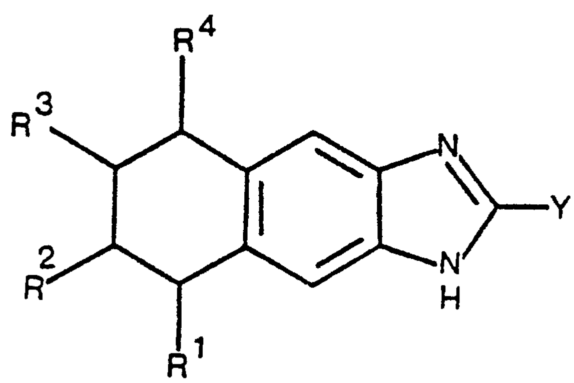

II

worin $R^1$, $R^2$, $R^3$ und $R^4$ obige und Y die unten angegebene Bedeutung besitzen, mit einer Verbindung der allgemeinen Formel

$$\overset{\displaystyle R^5}{\overset{|}{Y'-CH-X}} \qquad III$$

worin $R^5$ und X obige und Y' die unten angegebene Bedeutung besitzen,
umsetzt, wobei eines von Y und Y' eine Mercaptogruppe und das andere eine Abgangsgruppe bedeutet, oder

b) zur Herstellung von Verbindungen der Formel I, worin n die Zahl 1 bedeutet, eine entsprechende Verbindung der Formel I, worin n die Zahl 0 bedeutet, oxydiert,

erwünschtenfalls ein allfällig erhaltenes Diastereoisomerengemisch in die diastereoisomeren Racemate und/oder ein allfällig erhaltenes Racemat in die beiden Antipoden aufspaltet und/oder eine erhaltene freie Base in ein Säureadditionssalz und/oder ein erhaltenes Säureadditionssalz in die freie Base oder in ein anderes Säureadditionssalz überführt.

Nach einer ersten Ausführungsform des erfindungsgemässen Verfahrens wird somit eine Verbindung der allgemeinen Formel II mit einer Verbindung der allgemeinen Formel III umgesetzt, wobei entweder das Symbol Y in Formel II eine Mercaptogruppe und das Symbol Y' in Formel III eine Abgangsgruppe oder aber das Symbol Y in Formel II eine Abgangsgruppe und das Symbol Y' in Formel III eine Mercaptogruppe bedeuten. Abgangsgruppen sind beispielsweise Halogen, insbesondere Chlor, Brom oder Jod, oder Säurereste, z.B. der Rest einer starken organischen Sulfonsäure, z.B. ein Arylsulfonyloxyrest, wie Tosyloxy, oder ein Alkylsulfonyloxyrest, wie Mesyloxy. Weitere Beispiele von Abgangsgruppen sind Alkylmercaptoreste wie Methylmercapto, oder Alkylsulfinylreste wie Methylsulfinyl. Die Umsetzung der Verbindungen II und III erfolgt zweckmässigerweise in Gegenwart eines unter den Reaktionsbedingungen inerten Lösungsmittels oder Lösungsmittelgemisches und gegebenenfalls in Gegenwart einer Base. Als Basen eignen sich hierfür insbesondere anorganische Basen, wie Natrium- oder Kaliumhydroxid, Natrium- oder Kaliumhydrid und dergleichen oder organische Basen, wie Triäthylamin oder andere tertiäre Amine.

Als Lösungsmittel bzw. Lösungsmittelgemische eignen sich in erster Linie Alkohole, wie Aethanol, Gemische von Alkoholen und Wasser, Aether, wie Tetrahydrofuran, halogenierte Kohlenwasserstoffe, wie Methylenchlorid oder Chloroform. Ein bevorzugtes Lösungsmittel ist Dimethylformamid.

Die Reaktionstemperatur ist in ziemlich weiten Grenzen variierbar; sie wird in der Regel zwischen Raumtemperatur und der Siedetemperatur des Reaktionsgemisches liegen, wobei man vorzugsweise bei der Siedetemperatur des Reaktionsgemisches arbeitet. Eine zweckmässige Ausführungsform des vorliegenden Verfahrensaspektes besteht darin, dass man eine Verbindung der Formel II, worin Y eine Mercaptogruppe bedeutet, zunächst in ein Alkali-Derivat überführt, beispielsweise mittels Natriumhydroxyd, worauf dann die Umsetzung mit einer Verbindung der Formel III, worin Y, den Säurerest eines reaktionsfähigen Esters bedeutet, durchgeführt wird. In einer bevorzugten Ausführungsform nimmt man die Umsetzung in Dimethylformamid in Abwesenheit einer Base unter Erwärmen vor.

In einer weiteren Ausführungsform des erfindungsgemässen Verfahrens wird eine Verbindung der Formel I, worin n die Zahl 0 bedeutet, oxydiert. Dabei wird also ein Schwefelatom in die Sulfinylgruppe übergeführt, und man verwendet demgemäss hierfür Oxydationsmittel, welche für derartige Ueberführungen gebräuchlich sind, beispielsweise Persäuren, wie m-Chlorperbenzoesäure, Wasserstoffperoxyd, Perester, Natriummetaperjodat, Selendioxyd, Mangandioxyd usw. Die Oxydation erfolgt zweckmässigerweise in einem unter den Reaktionsbedingungen inerten organischen Lösungsmittel, beispielsweise in einem halogenierten Kohlenwasserstoff, wie Methylenchlorid, Chloroform, Dichloräthan und dergleichen oder in einem Kohlenwasserstoff, wie Benzol und dergleichen; bei Verwendung von Wasserstoffperoxyd als Oxydationsmittel kann auch in Wasser, Essigsäure und dergleichen gearbeitet werden. Es ist von Vorteil, das Oxydationsmittel in leichtem Ueberschuss bezüglich des zu oxydierenden Produktes einzusetzen. Bei der vorliegenden Ausführungsform des erfindungsgemässen Verfahrens wird zweckmässigerweise bei Raumtemperatur oder darunter gearbeitet.

Je nach der Struktur der Ausgangsprodukte und/oder der gewählten Ausführungsform des erfindungsgemässen Verfahrens können bestimmte Verbindungen der Formel I als optische Isomeren bzw. als Racemat oder, wenn sie wenigstens zwei asymmetrische Zentren enthalten, als Diastereoisomerengemische oder Racematgemische vorliegen. Erhaltene Diastereoisomerengemische und Racematgemische können auf Grund physikalisch-chemischer Unterschiede der Komponenten getrennt werden; Racemate können nach bekannten Methoden gespalten werden, beispielsweise durch fraktionierte Kristallisation von Salzen mit optisch aktiven Säuren.

Je nach den Verfahrensbedingungen und den eingesetzten Ausgangsmaterialien erhält man die Verbindungen der Formel I entweder als freie Basen oder als Säureadditionssalze. Die freien Basen können durch Umsetzung mit organischen oder anorganischen Säuren in entsprechende Salze übergeführt werden, wobei die Verwendung solcher Säuren bevorzugt ist, welche therapeutisch verträgliche Salze bilden, beispielsweise Chlorwasserstoff, Bromwasserstoff, Phosphorsäure, Schwefelsäure, Zitronensäure, Essigsäure, Bernsteinsäure, Maleinsäure, p-Toluolsulfonsäure und dergleichen. Die Säureadditionssalze der Verbindungen der Formel I, können in an sich bekannter Weise in die entsprechenden freien Basen oder in andere Säureadditionssalze übergeführt werden. Die Säureadditionssalze von Verbindungen der Formel I, worin n für die Zahl 1 steht, sind in wässeriger Lösung wenig stabil.

Die Imidazolderivate der Formel I und ihre Salze hemmen die Magensäuresekretion und die Entstehung von stress-induzierten Magenulcera.

Die Hemmung der Magensäuresekretion kann durch folgenden Versuch bestimmt werden: Hunden wird nach der Heidenhain-Technik [beschrieben von Rudick, J.Semb, L.S. und Nyhas, L.M.; J. Surgical Research 7: 383—398 (1967)] ein Teil des Magenfundus vom restlichen Magen in Form einer Tasche abgetrennt. In die Tasche wird eine Stahlkanüle eingenäht, die durch die Bauchdecke nach aussen geleitet wird. Nach Abheilen der Operationswunde kommen die Tiere in den Versuch, welcher an wachen Hunden durchgeführt wird. Die Magensekretion wird durch eine Infusion von 4-Methylhistamin-dihydrochlorid (40 $\mu$g/kg pro Std i.v.), einem selektiven Stimulator der Histamin $H_2$-Rezeptoren, angeregt. Sobald Volumen and pH der 15 Minuten-Fraktionen konstante Werte aufweisen, wird die Testsubstanz oral verabreicht. Diejenige Dosis der Prüfsubstanz wird ermittelt, die eine Hemmung der durch 4-Methylhistamin erzeugten Säuresekretion um 60—80% bewirkt.

Für das 2-[/(5-Methyl-2-pyridyl)methyl/thio]-1H-naphth[2,3-d]imidazol, welches an der Maus eine $DL_{50}$ von mehr als 8000 mg/kg p.o. zeigt, und für das 2-[(2-Pyridylmethyl)thio]-1H-naphth[2,3-d]-imidazol, welches an der Maus eine $DL_{50}$ von >5000 mg/kg p.o. zeigt, beträgt diese Dosis 3 mg/kg p.o.

Die Hemmung der Entstehung von stress-indizierten Magenulcera kann durch folgenden Versuch nachgewiesen werden: Unmittelbar nach oraler Applikation der Testsubstanz an weiblichen Ratten (Gewicht 140—160 g) werden die Tiere (10 Ratten pro Dosis) in ein Wasserbad (22,5°C; 7 cm hoch) gebracht. Nach 6 Stunden führt diese Stress-Situation in jedem unbehandelten Tier zu Magenulcera. Nach dieser definierten Zeit werden die behandelten Ratten getötet und diejenigen ohne Magenulcus gezählt. Die $ED_{50}$ — die Dosis, bei der 50% der Tiere gegen stress-induzierte Magenulcera geschützt sind — wird mittels der Probit-Methode bestimmt.

In diesem Test zeigt das 2-[/(5-Methyl-2-pyridylmethyl/thio]-1H-naphth[2,3-d]imidazol eine $ED_{50}$ von 31 mg/kg p.o. und das 2-[(2-Pyridylmethyl)thio]-1H-naphth[2,3-d]imidazol eine $ED_{50}$ von 12 mg/kg p.o.

Die Verbindungen der Formel I können daher als Heilmittel z.B. zur Behandlung von Magenulcera in Form pharmazeutischer Präparate Verwendung finden, welche sie oder ihre therapeutisch verträglichen Salze in Mischung mit einem für die enterale, perkutane oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutische wertvolle Stoffe enthalten.

Die Dosierung erfolgt nach individuellen Erfordernissen; bei peroraler Verabreichung ist in der Regel eine Tagesdosis von 100—400 mg und bei intravenöser Verabreichung eine Tagesdosis von 5—20 mg bevorzugt.

Die Ausgangsprodukte der Formeln II und III sind generell bekannt; sofern einzelne Verbindungen noch nicht beschrieben sind, können sie ohne Schwierigkeiten in Analogie zu den für die bekannten Verbindungen beschriebenen Darstellungsmethoden hergestellt werden.

In den nachfolgenden Beispielen, welche die vorliegende Erfindung illustrieren, deren Umfang jedoch in keiner Weise beschränken sollen, sind sämtliche Temperaturen in °C angegeben.

Beispiel 1

In einem 250 ml Sulfierkolben mit Rührer, Thermometer, Kühler und Tropftrichter wurden 10,1 g 1H-Naphth[2,3-d]imidazol-2-thiol in 100 ml Alkohol suspendiert. Dann tropfte man eine Lösung von 4,0 g Natriumhydroxyd in 50 ml Wasser zu, wobei eine klare Lösung entstand. Hierauf erhitzte man auf Siedetemperatur, gab 8,2 g 2-Chlormethyl-pyridin-hydrochlorid zu und liess über Nacht am Rückfluss kochen. Das Reaktionsgemisch wurde anschliessend eingedampft, der Rückstand in 600 ml Essigester und 200 ml Wasser aufgenommen und die Essigester-Phase zweimal mit 200 ml Wasser gewaschen, über Natriumsulfat getrocknet und im Vakuum eingedampft. Der Rückstand wurde aus Essigester/Petroläther einmal umkristallisiert und lieferte 2-[(2-Pyridylmethyl)thio]-1H-naphth[2,3-d]-imidazol vom Schmelzpunkt 165—167°; das Hydrochlorid schmilzt bei 230—231°.

*Herstellung der Ausgangsstoffe:*

125,0 g 2,3-Dihydroxynaphthalin wurden in 3,5 l $NH_3$ (25%ig) suspendiert und bei 240°/30 bar $N_2$ 60 Stunden geschüttelt. Die Suspension wurde abgenutscht, mit 1,5 l Wasser gewaschen und dann in 7 l Essigester gelöst. Die Essigesterlösung wurde zweimal mit je 1 l 3N NaOH und zweimal mit je 1 l $H_2O$ extrahiert, über $Na_2SO_4$ getrocknet, durch Kohle filtriert und das entfärbte Filtrat im Vakuum bei 40° eingedampft. Der Rückstand wurde 1 Stunde lang bei Raumtemperatur in 400 ml Acetonitril gerührt, abgenutscht und getrocknet. 128 g so erhaltenes 2,3-Diaminonaphthalin wurden in 900 ml Aethanol suspendiert. Unter intensivem Rühren tropfte man eine Lösung von 51,0 g KOH in 160 ml Wasser hinzu. Nach 10 Minuten Rühren bei Zimmertemperatur wurden 71,6 g Schwefelkohlenstoff zugetropft. Das Reaktionsgemisch rührte man 1 Stunde lang bei ca. 20°, dann über Nacht bei Siedetemperatur weiter. Nach der Zugabe von 91,0 g KOH in 370 ml Wasser ging alles in Lösung. Man nutscht durch 50 g Kohle und verdünnte das Filtrat mit 900 ml Wasser. Bei 60—70° Innentemperatur und unter Rühren wurde 295 ml Eisessig in 295 ml Wasser tropfenweise zugegeben. Anschliessend wurde die Suspension noch 1 Stunde bei gleicher Temperatur gerührt, dann im Eisbad abgekühlt und genutscht. Der Rückstand wurde mit 300 ml Wasser und darauf mit 100 ml Aethanol gewaschen. Nach der Trocknung im Vakuum bei 60° erheilt man 158,0 g Rohprodukt, das in 600 ml Dioxan suspendiert bei ca. 20° l Stunde lang gerührt und dann abgenutscht wurde. Der Niederschlag wurde nacheinander mit 100 ml Dioxan und 300 ml Aether gewaschen. Man erhielt nach Trocknung im Vakuum bei 60° 148,1 g 1H-Naphth[2,3-d]imidazol-2-thiol. Smp. 303—305°.

120 g 2-Hydroxymethylpyridin wurden in 2 l abs. Benzol gerührt. Bei 0—5° tropfte man 100 ml Thionylchlorid, langsam hinzu und rührte das Reaktionsgemisch über Nacht bei ca. 20°. Es wurde auf 0—5° abgekühlt und abgenutscht. Der Niederschlag wurde in 1000 ml Aethanol heiss gelöst, über Kohle filtriert und bei 50° eingedampft. Den Rückstand löste man in 400 ml Acetonitril, rührte bei Raumtemperatur 1 Stunde und nutscht das 2-Chlormethylpyridin-HCl ab. Schmelzpunkt 121—123°.

Beispiel 2

In einem 1 Liter Vierhalsrundkolben mit Rührer, Thermometer, Tropftrichter und Chlorcalciumrohr wurden 9,4 g 2-[(2-Pyridylmethyl)thio]-1H-naphth[2,3-d]imidazol in 250 ml Methylenchlorid suspendiert. Unter heftigen Rühren tropfte man unter Eis/Methanol-Kühlung eine Lösung von 6,7 g m-Chlorperbenzoesäure in 150 ml Methylenchlorid zu und rührte das Gemisch anschliessend noch 5—7 Stunden bei 0—5°. Dann wurde mit dreimal 100 ml Natriumbicarbonat-Lösung gewaschen, mit zweimal 200 ml Kochsalz-Lösung neutral gewaschen, über Natriumsulfat getrocknet und im Vakuum eingedampft. Der Rückstand wurde aus 500 ml Toluol umkristallisiert und lieferte 2-[(2-Pyridylmethyl)-sulfinyl]-1H-naphth[2,3-d]imidazol vom Schmelzpunkt 145—146°.

Beispiel 3

0,92 g 2-Chlor-1H-naphth[2,3-d]imidazol und 0,57 g 2-Mercaptomethylpyridin wurden in 15 ml Aethanol und 4,5 ml 1N Natronlauge 18 Stunden zum Rückfluss erhitzt. Die Reaktionslösung wurde mit 50 ml Wasser versetzt und zweimal mit total 50 ml Essigsäureäthylester extrahiert. Die organischen Phasen wurden mit gesättigter Kochsalzlösung gewaschen, über Magnesiumsulfat getrocknet und am Rotationsverdampfer zur Trockene eingeengt. Kristallisation aus 50 ml Acetonitril lieferte 0,68 g 2-[(2-Pyridylmethyl)thio]-1H-naphth[2,3-d]imidazol vom Smp. 169—170°.

*Herstellung des Ausgangsstoffs:*

16,2 g 2-Hydroxy-1H-naphth[2,3-d]imidazol wurden in 170 ml Phosphoroxychlorid 3 Stunden zum Rückfluss erhitzt. Die Reaktionslösung wurde eingeengt, mit Wasser zersetzt und mit Ammoniak basisch gestellt. Der abgenutschte Rückstand wurde an 500 g Silicagel chromatographiert. Essigsäureäthylester eluierte 2,5 g 2-Chlor-1H-naphth[2,3-d]imidazol. Kristallisation aus Methanol lieferte ein Produkt mit Smp. >300°.

Beispiel 4

0,45 g 2-Methylthio-1H-naphth[2,3-d]imidazol und 0,65 g 2-Mercaptomethylpyridin wurden in 10 ml Aethanol 40 Stunden zum Rückfluss erhitzt. Zur homogenen Lösung wurden 10 ml Wasser gegeben und der entstandene Niederschlag abfiltriert. Man erhielt 0,50 g 2-[(2-Pyridylmethyl)thio]-1H-naphth[2,3-d]imidazol vom Smp. 170°.

*Herstellung des Ausgangsstoffs:*

20,0 g 1H-Naphth[2,3-d]imidazol-2-thiol und 4,0 g Natriumhydroxid wurden in 150 ml Aethanol mit 6,5 g Methyljodid 1 Stunde zum Rückfluss erhitzt. Die Reaktionslösung wurde mit 500 ml Wasser verdünnt und das ausgefallene Produkt abgenutscht. Kristallisation aus Dioxan lieferte 12,0 g 2-Methylthio-1H-naphth[2,3-d]imidazol vom Smp. 242—244°.

Beispiel 5

0,165 g 2-(Methylsulfinyl)-1H-naphth[2,3-d]imidazol und 0,090 g 2-Mercaptomethylpyridin wurden in 5 ml Aethanol und 0,72 ml 1N Natronlauge 1 Stunde zum Rückfluss erhitzt. Die Reaktionslösung wurde eingeengt und an Silicagel chromatographiert. Essigsäureäthylester eluierte 0,050 g 2-[(2-Pyridylmethyl)thio]-1H-naphth[2,3-d]imidazol.

*Herstellung des Ausgangsstoffs:*

4,3 g 2-(Methylthio)-1H-naphth[2,3-d]imidazol in 500 ml Methylenchlorid wurde bei 0—5° mit einer Lösung von 4,2 g m-Chlorperbenzoesäure in 100 ml Methylenchlorid tropfenweise versetzt. Die Reaktionslösung wurde 90 Minuten bei 0° gerührt, zweimal mit wässeriger Kaliumbicarbonatlösung und einmal mit gesättigter Natriumchloridlösung gewaschen, über Magnesiumsulfat getrocknet und am

Rotationsverdampfer eingeengt. Kristallisation aus Acetonitril lieferte 2,3 g 2-(Methylsulfinyl)-1H-naphth[2,3-d]imidazol vom Smp. 194°.

Beispiel 6

90,0 g 1H-Naphth[2,3-d]imidazol-2-thiol in 200 ml Dimethylformamid wurden auf 95° erhitzt. Zu der homogenen Lösung wurden 80,1 g 2-Chlormethyl-5-methylpyridin-hydrochlorid zugegeben und 10 Minuten bei 95° gehalten. Nach dem Abkühlen auf Raumtemperatur wurde 1 l Aether zugemischt und abgenutscht. Der Rückstand wurde in 2N Kaliumbicarbonatlösung aufgenommen und mit total 2 l Methylenchlorid extrahiert. Die organischen Phasen wurden mit gesättigter Kochsalzlösung gewaschen, über Magnesiumsulfat getrocknet und anschliessend am Rotationsverdampfer eingeengt. Der Rückstand wurde aus 2 l Alkohol umkristallisiert. Man erhielt 106 g 2-/[(5-Methyl-2-pyridyl)methyl]-thio/-1H-naphth[2,3-d]imidazol vom Smp. 185—186°.

*Herstellung des 2-Chlormethyl-5-methylpyridin-hydrochlorids:*

11,5 g 2,5-Dimethylpyridin-N-oxid in 5 ml Essigsäure wurden tropfenweise zu auf 120° aufgewärmten 18 ml Essigsäureanhydrid gegeben. Die Reaktionslösung wurde noch 45 Minuten am Rückfluss gekocht, am Rotationsverdampfer eingeengt und bei 10,7mbar/115° destilliert. Man erhielt 14,0 g 2-Acetoxymethyl-5-methylpyridin.

14,0 g 2-Acetoxymethyl-5-methylpyridin wurden in einer Lösung von 4,5 g Natriumhydroxid in 150 ml Wasser 90 Minuten zum Rückfluss erhitzt. Die abgekühlte Lösung wurde mit Natriumchlorid gesättigt und mit total 100 ml Chloroform extrahiert. Die organischen Phasen wurden mit gesättigter Natriumchloridlösung gewaschen, über Magnesiumsulfat getrocknet und bei 40°/21,3 mbar eingeengt. Das erhaltene Oel destilliert bei 12°/10,7 mbar. Man erhielt 8,7 g 2-Hydroxymethyl-5-methylpyridin.

8,7 g 2-Hydroxymethyl-5-methyl-pyridin wurden bei einer Reaktionstemperatur von 0—10° tropfenweise zu 18 ml Thionylchlorid gegeben. Die Reaktionslösung wurde anschliessend 15 Stunden bei Raumtemperatur gerührt und dann am Rotationsverdampfer eingeengt. Der Rückstand wurde in Alkohol mit Aktivkohle behandelt und aus Alkohol-Aether kristallisiert. Man erhielt 11,5 g 2-Chlormethyl-5-methylpyridin-hydrochloride vom Smp. 145—146°.

Beispiel 7

8,1 g 1H-Naphth[2,3-d]imidazol-2-thiol in 80 ml Aethanol und 40 ml Wasser wurden mit 3.2 g Natriumhydroxid und 7,2 g 2-Chlormethyl-4-methyl-pyridin-hydrochlorid versetzt und 2 Stunden zum Rückfluss erhitzt. Die Reaktionslösung wurde eingeengt und zwischen Wasser und Essigsäureäthylester/Tetrahydrofuran verteilt. Die organischen Phasen wurden mit gesättigter Kochsalzlösung gewaschen, über Magnesiumsulfat getrocknet und am Rotationsverdampfer zur Trockene eingeengt. Der ölige Rückstand wurde aus Toluol kristallisiert. Man erhielt 6,5 g 2-/[(4-Methyl-2-pyridyl)methyl]thio/-1H-naphth[2,3-d]imidazol vom Smp. 170—172°.

Analog wurde 2-/[(6-Methyl-2-pyridyl)methyl]thio/-1H-naphth[2,3-d]imidazol, Smp. 119—120° hergestellt.

Beispiel 8

Zu 8 g 1H-Naphth[2,3-d]imidazol-2-thiol in 80 ml Aethanol wurden 6,4 g NaOH in 40 ml Wasser bei 0° zugegeben und anschliessend unter Rühren 8,72 g rac-2-(1-Chloräthyl)-pyridin in 40 ml Aethanol zugetropft. Nach 12 Stunden Kochen am Rückfluss wurde die Lösung im Vakuum eingedampft und der Rückstand mit Essigester-Wasser nochmals aufgearbeitet. Aus 11,5 g Rohprodukt erhielt man nach Reinigung an Silicagel und Kristallisation aus Essigester 6,0 g rac-2-/[1-(2-Pyridyl)äthyl]thio/-1H-naphth[2,3-d]imidazol, Smp. 129—133°.

Die optische Spaltung des Racemats wurde mittels (+) bzw. (—)-Di-O,O'-p-toluoyl-weinsäure (DITTA) in Essigester-Toluol (1:1) ausgeführt.

(—) (RR) DITTA-Salz mit (+) Base Smp. 142—143°, $[\alpha]_D$ = —93,7° (in Methanol, c = 1,0)

(+) Base Smp. 136—137°, $[\alpha]_D$ = +413,8°

(+) (SS) DITTA-Salz mit (—) Base Smp. 136—137°, $[\alpha]_D$ = —92,1°

(—) Base Smp. 135,5—136,5°, $[\alpha]_D$ = —408,6°

Beispiel 9

10,0 g 1H-Naphth[2,3-d]imidazol-2-thiol und 9,6 g 2-Chlormethyl-5-äthyl-pyridin-hydrochlorid wurden in 200 ml Aethanol und 100 ml 1N Natronlauge 2 Stunden zum Rückfluss erhitzt. Die Reaktionslösung wurde am Rotationsverdampfer eingeengt, in 250 ml Wasser aufgenommen und dreimal mit 100 ml Essigsäureäthylester extrahiert. Die organischen, Phasen wurden mit gesättigter Koch salzlösung gewaschen, über Magnesiumsulfat getrocknet und zur Trockene eingeengt. Kristallisation aus Acetonitril lieferte 8,2 g 2-/[(5-Aethyl-2-pyridyl)methyl]thio/-1H-naphth[2,3-d]imidazol vom Smp. 156—157°.

Beispiel 10

Zu 4,09 g 5,6,7,8-Tetrahydro-1H-naphth[2,3-d]imidazol-2-thiol in 50 ml Aethanol wurden 2,24 g

KOH in 10 ml Wasser, dann 3,28 g 2-Chlormethylpyridin. HCl in 20 ml Aethanol zugegeben. Nach 12 Stunden Kochen unter Rückfluss dampfte man die Lösung im Vakuum ein und arbeitete den Rückstand mit Essigester-Wasser normal auf. Die auf ca. 50 ml eingeengte und auf 0° abgekühlte Essigester-Phase ergab 4,8 g Kristalle vom Smp. 95—97°, die aus 50 ml Acetonitril umkristallisiert wurden: 4,15 g 5,6,7,8-Tetrahydro-2-[(2-pyridylmethyl)thio]-1H-naphth[2,3-d]imidazol, Smp. 98—101°.

*Herstellung des Ausgangsstoffs:*

Zu einer Lösung von 16,2 g 5,6,7,8-Tetrahydro-2,3-diaminonaphthalin in 100 ml Isopropanol und 20 ml Aethanol wurden zuerst bei 0° 6,16 g KOH in 20 ml Wasser gelöst, dann 7,3 ml Schwefelkohlenstoff zugetropft. Nach 2 Stunden Kochen am Rückfluss wurde das pH mit Eisessig auf 3—4 gestellt und die ausgefallenen weissen Kristalle bei 0° genutscht. Die Kristalle wurden in 200 ml Isopropanol gelöst, die Lösung ein halbe Stunde unter Rückfluss gekocht, und bei 0° abgenutscht. Man erhielt 17,8 g 5,6,7,8-Tetrahydro-1H-naphth[2,3-d]imidazol-2-thiol, Smp. 282—289°.

## Beispiel 11

3,2 g 2-/[(5-Aethyl-2-pyridyl)methyl]thio/-1H-naphth[2,3-d]imidazol wurden in 200 ml Methylenchlorid bei einer Reaktionstemperatur von 0—5° tropfenweise mit einer Lösung von 1,7 g m-Chlorperbenzoesäure in 50 ml Methylenchlorid versetzt. Die Reaktionslösung wurde bei 0° 3 Stunden gerührt, dann zweimal mit wässriger Kaliumbicarbonatlösung, einmal mit gesättigter Kochsalzlösung gewaschen und anschliessend zur Trockene eingeengt. Der grüne Rückstand wurde in Acetonitril/Methanol mit Aktivkohle behandelt und aus demselben Lösungsmittelgemisch kristallisiert. Man erhielt 2,1 g 2-/[(5-Aethyl-2-pyridyl)methyl]sulfinyl/-1H-naphth[2,3-d]imidazol vom Smp. 177°.

## Beispiel 12

1,1 g 2-Hydroxymethylthiazol wurden in 50 ml Chloroform gelöst und mit 0,82 ml Thionylchlorid 2 Stunden bei 20° gerührt, dann 30 Minuten unter Rückfluss gekocht. Nach Eindampfen im Vakuum bei 40° nahm man den Rückstand in 20 ml Aethanol auf und dampfte die Lösung erneut ist. Die erhaltenen Kristalle von Chlormethylthiazol-hydrochlorid wurden mit einer Lösung von 1,91 g 1H-Naphth[2,3-d]-imidazol-2-thiol in 50 ml Aethanol, 2,14 g KOH und 10 ml Wasser versetzt und 2 Stunden am Rückfluss gekocht. Das Lösungsmittel wurde im Vakuum entfernt und der Rückstand mit Essigester-Wasser aufgearbeitet. Nach zweimaliger Umkristallisation aus Acetonitril erhielt man 1,3 g 2-[(2-Thiazolylmethyl)thio]-1H-naphth[2,3-d]imidazol, Smp. 153—157°.

## Beispiel 13

1,0 g 1H-Naphth[2,3-d]imidazol-2-thiol und 0,8 g 2-Chlormethylimidazol-hydrochlorid wurden in 20 ml Dimethylformamid 1 1/2 Stunden auf 90° erhitzt. Der entstandene Niederschlag wurde abfiltriert, mit wenig Acetonitril gewaschen und in 2N Natriumbicarbonatlösung gelöst. Die wässrige Phase wurde dreimal mit 30 ml Essigsäureäthylester extrahiert. Die organischen Phasen wurden mit gesättigter Kochsalzlösung gewaschen, über Magnesiumsulfat getrocknet und anschliessend eingeengt. Der Rückstand wurde aus 150 ml Acetonitril umkristallisiert. Man erhielt 150 mg 2-[(Imidazol-2-ylmethyl)-thio]-1H-naphth[2,3-d]imidazol, Smp. 172°.

## Beispiel 14

10,0 g 1H-Naphth[2,3-d]imidazol-2-thiol und 8,0 g 2-Chlormethylimidazolin-hydrochlorid wurden in 50 ml Dimethylformamid für 3 Stunden auf 90° erhitzt. Nach Abkühlen auf Raumtemperatur wurde mit 200 ml Toluol verdünnt. Der Rückstand wurde abgenutscht, gut mit Toluol gewaschen, und aus 700 ml Methanol unter Zugabe von 300 ml Aether kristallisiert. Man erhielt 10,3 g 2-[(2-Imidazolin-2-ylmethyl)thio]-1H-naphth[2,3-d]imidazol-dihydrochlorid vom Smp. 227—228°.

## Beispiel 15

2,5 g 2-(1-Hydroxyäthyl)-thiazol wurden in 50 ml abs. Chloroform mit 2,38 g Thionylchlorid in 10 ml Chloroform bei 0° versetzt, und 1 Stunde bei 0°, 30 Minuten bei 20° und 1 Stunde unter Rückfluss gerührt. Nach Entfernen des Lösungsmittels im Vakuum löste man den Rückstand in 20 ml Aethanol auf und dampfte die Lösung wieder ein. Zum zurückgebliebenen Oel wurde das Gemisch von 4,0 g 1H-Naphth[2,3-d]imidazol-2-thiol, 80 ml Aethanol, 4,48 g KOH und 40 ml Wasser zugegeben und 12 Stunden unter Rückfluss gekocht. Nach dem Eindampfen im Vakuum nahm man den Rückstand in 300 ml Wasser auf und extrahierte ihn dreimal mit 200 ml Methylenchlorid. Die zur Trockene eingedampften organischen Phasen kristallisierten aus Benzol. Nach zweimaliger Umkristallisation aus Benzol erhielt man 1,8 g 2-/[1-(2-Thiazolyl)äthyl]thio/-1H-naphth[2,3-d]imidazol, Smp. 184—186°.

## Beispiel 16

2,85 g 5,6,7,8-Tetrahydro-1H-naphth[2,3-d]imidazol-2-thiol wurden zusammen mit 50 ml Aethanol, 2,24 g KOH, 10 ml Wasser und 2,5 g 2-Chlormethyl-5-methyl-pyridin 12 Stunden unter Rückfluss gekocht, dann im Vakuum eingedampft und mit Methylenchlorid-Wasser aufgearbeitet. Aus 4,8 g

Rückstand erhielt man nach Umkristallisation aus 80 ml Acetonitril 3,78 g 5,6,7,8-Tetrahydro-2-/[(5-methyl-2-pyridyl)methyl]thio/-1H-naphth[2,3-d]imidazol vom Smp. 135—136°.

Analog wurde 5,6,7,8-Tetrahydro-2-/[1-(2-pyridyl)äthyl]thio/-1H-naphth[2,3-d]imidazol hergestellt, Smp. 124—125°.

Beispiel 17

3,05 g 2-/[(5-Methyl-2-pyridyl)methyl]thio/-1H-naphth[2,3-d]imidazol und 3,0 g Kaliumcarbonat wurden in 300 ml Methylenchlorid bei 0—5° mit einer Lösung von 1,725 g m-Chlorperbenzoesäure in 50 ml Methylenchlorid versetzt. Anschliessend wurde bei 0° 30 Minuten gerührt, dann mit wässriger Kaliumbicarbonatlösung und gesättigter Kochsalzlösung gewaschen, über Magnesiumsulfat getrocknet und bei 40°/21,3 mbar eingedampft. Der Rückstand wurde zweimal aus Acetonitril umkristallisiert. Man erhielt 1,6 g 2-/[(5-Methyl-2-pyridyl)methyl]sulfinyl/-1H-naphth[2,3-d]imidazol vom Smp. 181°.

Analog wurde 5,6,7,8-Tetrahydro-2-[(2-pyridylmethyl)sulfinyl]-1H-naphth[2,3-d]imidazol, Smp. 155—156°, hergestellt.

Beispiel 18

8,0 g 1H-Naphth[2,3-d]imidazol-2-thiol wurden in 20 ml Dimethylformamid bei 90° mit 6,7 g 4-Chlormethyl-5-methyl-imidazol-hydrochlorid versetzt. Nach 10 Minuten wurde auf Raumtemperatur abgekühlt und mit Aether versetzt. Der Niederschlag wurde abgenutscht und aus Wasser-Aethanol umkristallisiert. Man erhielt 6,5 g 2-/[(5-Methylimidazol-4-yl)methyl]thio/-1H-naphth[2,3-d]imidazol-dihydrochlorid vom Smp. 265—267°.

Beispiel 19

Zu 6,2 g 5,6,7,8-Tetrahydro-2-/[1-(2-pyridyl)äthyl/-thio/-1H-naphth[2,3-d]imidazol in 50 ml Methylenchlorid wurden bei 0° 35 ml 10%iger Lösung von m-Chlorperbenzoesäure zugetropft. Nach 3 Stunden Rühren bei 0° wurde das ausgefallene Material abgenutscht, mit Acetonitril aufgeschlämmt, wieder genutscht und mit Aether, anschliessend mit Petroläther gewaschen. Man erhielt 2,7 g 5,6,7,8-Tetrahydro-2-/[1-(2-pyridyl)äthyl]sulfinyl/-1H-naphth[2,3-d]imidazol, Smp. 152—153°.

Analog wurden hergestellt:

5,6,7,8-Tetrahydro-2-/[(5-methyl-2-pyridyl)methyl]sulfinyl/-1H-naphth[2,3-d]imidazol, Smp. 188—189°,

2-/[1-(2-Pyridyl)äthyl]sulfinyl/-1H-naphth[2,3-d]imidazol, Smp. 172—173°.

Beispiel A

Man stellt Tabletten der folgenden Zusammensetzung her:

| | |
|---|---|
| 2-/[(5-Methyl-2-pyridyl)methyl]thio/-1H-naphth[2,3-d]imidazol | 50,0 mg |
| Milchzucker pulv. | 100,0 mg |
| Maisstärke weiss | 48,0 mg |
| Magnesiumstearat | 2,0 mg |
| | 200,0 mg |

Die Mischung von feingemahlenem Wirkstoff, Milchzucker und einem Teil der Maisstärke wird mit einem Kleister aus Wasser und einem zweiten Teil der Maisstärke geknetet, granuliert, getrocknet und gesiebt. Das Granulat wird dann zuerst mit dem Rest der Maisstärke und darauf mit dem Magnesiumstearat gemischt. Die Mischung wird zu Tabletten zu 200 mg verpresst.

Beispiel B

Man stellt Tabletten der folgenden Zusammensetzung her:

| | |
|---|---|
| 2-[(2-Pyridylmethyl)thio]-1H-naphth[2,3-d]imidazol | 100,0 mg |
| Milchzucker pulv. | 150,0 mg |
| Maisstärke weiss | 145,0 mg |
| Magnesiumstearat | 5,0 mg |
| | 400,0 mg |

Die Mischung von feingemahlenem Wirkstoff, Milchzucker und einem Teil der Maisstärke wird mit einem Kleister aus Wasser und einem zweiten Teil der Maisstärke geknetet, granuliert, getrocknet und gesiebt. Das Granulat wird zuerst mit dem Rest der Maisstärke und darauf mit dem Magnesiumstearat gemischt. Die Mischung wird zu Tabletten à 400 mg verpresst.

## Patentansprüche

1. Imidazolderivate der allgemeinen Formel

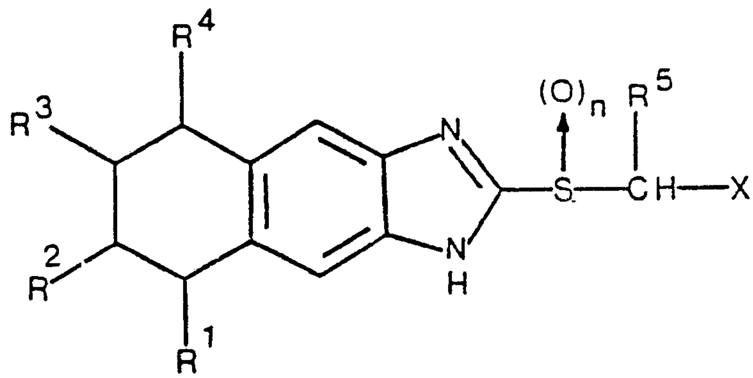

I

worin $R^1$, $R^2$, $R^3$ und $R^4$ je Wasserstoff oder $R^1$ zusammen mit $R^2$ und $R^3$ zusammen mit $R^4$ je eine zusätzliche Kohlenstoff-Kohlenstoff-Bindung, n die Zahl 0 oder 1, $R^5$ Wasserstoff oder niederes Alkyl und X einen gegebenenfalls durch niederes Alkyl monosubstituierten 2-Pyridylrest, einen 2-Imidazolylrest, einen 2-Imidazolinylrest, einen 2-Thiazolylrest, einen 2-Thiazolinylrest, oder einen gegebenenfalls durch niederes Alkyl monosubstituierten 4(5)-Imidazolylrest bedeuten, und deren Säureadditionssalze.

2. Verbindungen gemäss Anspruch 1, worin X einen gegebenenfalls durch niederes Alkyl monosubstituierten 2-Pyridylrest, einen 2-Imidazolylrest, einen 2-Imidazolinylrest, einen 2-Thiazolylrest oder einen 2-Thiazolinylrest bedeutet.

3. Verbindungen gemäss Anspruch 2, worin X einen gegebenenfalls durch niederes Alkyl monosubstituierten 2-Pyridylrest bedeutet.

4. Verbindungen gemäss Anspruch 3, worin X einen Methyl-substituierten 2-Pyridylrest bedeutet.

5. Verbindungen gemäss Anspruch 4, worin die Methylgruppe 5- oder 6-ständig ist.

6. Verbindungen gemäss einem der Ansprüche 1 bis 5, worin $R^5$ Wasserstoff bedeutet.

7. Verbindungen gemäss einem der Ansprüche 1 bis 6, worin $n = 0$ ist.

8. Verbindungen gemäss einem der Ansprüche 1 bis 7, worin $R^1$ zusammen mit $R^2$ und $R^3$ zusammen mit $R^4$ je eine zusätzliche Kohlenstoff-Kohlenstoff-Bindung bedeuten.

9. 2-/[(5-Methyl-2-pyridyl)methyl]thio/-1H-naphth[2,3-d]imidazol und dessen Säureadditionssalze.

10. 2-[(2-Pyridylmethyl)thio]-1H-naphth[2,3-d]imidazol und dessen Säureadditionssalze.

11. Verfahren zur Herstellung von Imidazolderivaten der in Anspruch 1 definierten allgemeinen Formel I und ihrer Säureadditionssalze, dadurch gekennzeichnet, dass man

a) zur Herstellung von Verbindungen der Formel I, worin n die Zahl 0 bedeutet, eine Verbindung der allgemeinen Formel

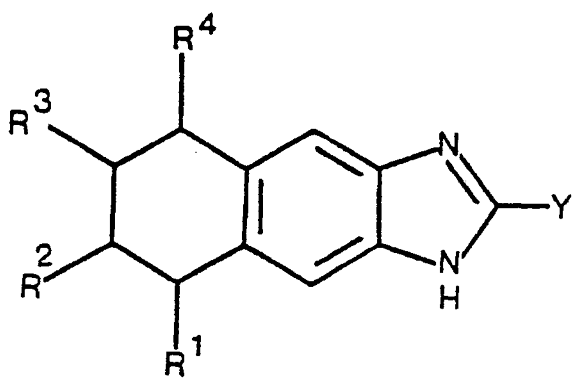

II

worin $R^1$, $R^2$, $R^3$ und $R^4$ obige und Y die unten angegebene Bedeutung besitzen, mit einer Verbindung der allgemeinen Formel

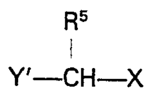

III

worin $R^5$ und X obige und Y' die unten angegebene Bedeutung besitzen, umsetzt, wobei eines von Y und Y' eine Mercaptogruppe und das andere eine Abgangsgruppe bedeutet, oder

b) zur Herstellung einer Verbindung der Formel I, worin n die Zahl 1 bedeutet, eine entsprechende Verbindung der Formel I, worin n die Zahl 0 bedeutet, oxydiert, erwünschtenfalls ein allfällig erhaltenes Diastereoisomerengemisch in die diastereoisomeren Racemate und/oder ein allfällig erhaltenes Racemat in die beiden Antipoden aufspaltet und/oder erwünschtenfalls eine erhaltene freie Base in ein Säureadditionssalz und/oder ein erhaltenes Säureadditionssalz in die freie Base oder in ein anderes Säureadditionssalz überführt.

12. Verfahren nach Anspruch 11, dadurch gekennzeichnet, dass man 2-/[(5-Methyl-2-pyridyl)-methyl]thio/-1H-naphth[2,3-d]imidazol oder ein Säureadditionssalz davon herstellt.

13. Verfahren nach Anspruch 11, dadurch gekennzeichnet, dass man 2-[(2-Pyridylmethyl)thio]-1H-naphth[2,3-d]imidazol oder ein Säureadditionssalz davon herstellt.

14. Pharmazeutische Präparate, enthaltend ein Imidazolderivat der in Anspruch 1 definierten allgemeinen Formel I oder ein Säureadditionssalz davon.

**Revendications**

1. Dérivés imidazoliques de formule générale

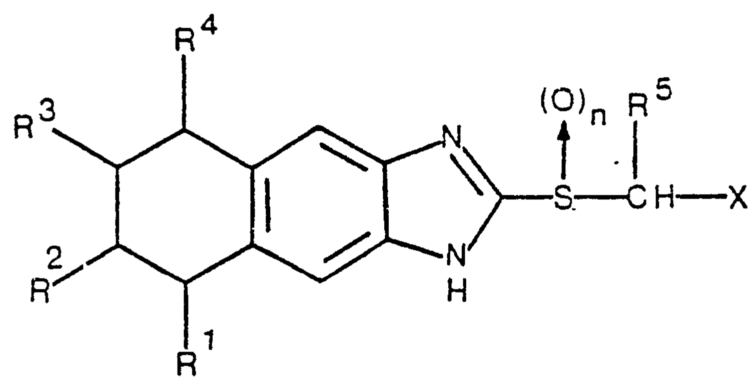

I

dans laquelle $R^1$, $R^2$, $R^3$ et $R^4$ représentent chacun un hydrogène ou $R^1$ représente avec $R^2$, et $R^3$ avec $R^4$, respectivement, une liaison carbone-carbone supplémentaire, n représente le nombre 0 ou 1, $R^5$ un hydrogène ou un alcoyle inférieur et X un radical 2-pyridyle éventuellement mono-substitué par un alcoyle inférieur, un radical 2-imidazolyle, un radical 2-imidazolinyle, un radical 2-thiazolyle, un radical 2-thiazolinyle, ou un radical 4(5)-imidazolyle éventuellement mono-substitué par un alcoyle inférieur, et leurs sels d'addition d'acides.

2. Composés selon la revendication 1, où X représente un radical 2-pyridyle éventuellement mono-substitué par un alcoyle inférieur, un radical 2-imidazolyle, un radical 2-imidazolinyle, un radical 2-thiazolyle ou un radical 2-thiazolinyle.

3. Composés selon la revendication 2, où X représente un radical 2-pyridyle éventuellement mono-substitué par un alcoyle inférieur.

4. Composés selon la revendication 3, où X représente un radical 2-pyridyle substitué par un méthyle.

5. Composés selon la revendication 4, où le groupe méthyle est en position 5 ou 6.

6. Composés selon l'une des revendications 1 à 5, où $R^5$ représente un hydrogène.

7. Composés selon l'une des revendications 1 à 6, où $n = 0$.

8. Composés selon l'une des revendications 1 à 7, où $R^1$ représente avec $R^2$, et $R^3$ avec $R^4$, respectivement, une liaison carbone-carbone supplémentaire.

9. Le 2-/[(5-méthyl-2-pyridyl)méthyl]thio/-1H-napht-[2,3-d]imidazole et ses sels d'addition d'acides.

10. Le 2-[(2-pyridylméthyl)thio]-1H-napht[2,3-d]imidazole et ses sels d'addition d'acides.

11. Procédé de préparation de dérivés imidazoliques de formule générale I définie dans la revendication 1 et de leurs sels d'addition d'acides, caractérisé en ce que:

a) pour préparer les composés de formule I où n représente le nombre 0, on fait réagir un composé de formule générale

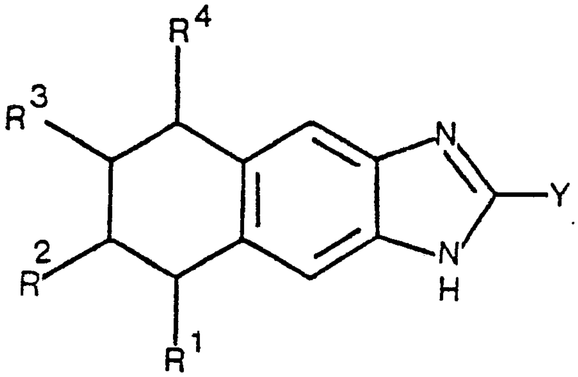

II

où $R^1$, $R^2$, $R^3$ et $R^4$ ont la signification donnée ci-dessus et où Y a la signification donnée ci-dessous avec un composé de formule générale

$$\begin{array}{c} R^5 \\ | \\ Y'—CH—X \end{array}$$ III

où $R^5$ et X ont la signification donnée ci-dessus et Y' a la signification donnée ci-dessous, l'un des groupes Y et Y' représentant un groupe mercapto en l'autre un groupe sortant, ou

b) pour préparer les composés de formule I où n représente le nombre 1, on oxyde un composé de formule I correspondant où n représente le nombre 0, le cas échéant, on résoud un mélange de diastéréoisomères éventuellement obtenu en les racémates diastéréoisomériques, et/ou un racémate éventuellement obtenu en les deux antipodes, et/ou on transforme une base libre obtenue en un sel d'addition d'acide et/ou un sel d'addition d'acide obtenu en la base libre ou en un autre sel d'addition d'acide.

12. Procédé selon la revendication 11, caractérisé en ce qu'on prépare le 2-/[(5-méthyl-2-pyridyl)méthyl]thio/-1H-napht[2,3-d]imidazole ou un de ses sels d'addition d'acides.

13. Procédé selon la revendication 11, caractérisé en ce qu'on prépare le 2-[(2-pyridylméthyl)-thio]-1H-napht[2,3-d]imidazole ou un de ses sels d'addition d'acides.

14. Préparations pharmaceutiques contenant un dérivé d'imidazole de formule générale I définie dans la revendication 1 ou un de ses sels d'addition d'acides.

**Claims**

1. Imidazole derivatives of the general formula

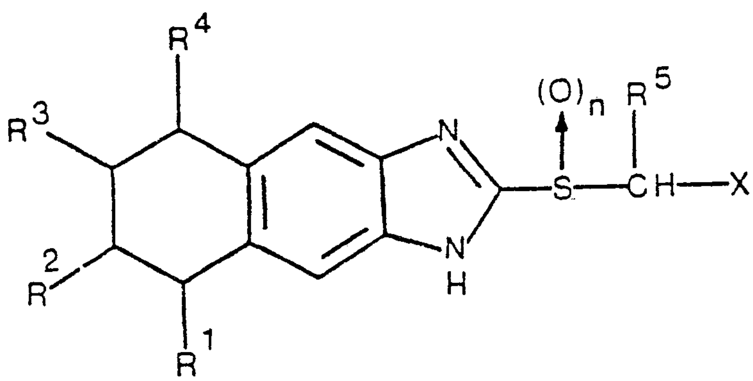

I

wherein $R^1$, $R^2$, $R^3$ and $R^4$ each signify hydrogen or $R^1$ together with $R^2$ and $R^3$ together with $R^4$ each signify an additional carbon-carbon bond, n signifies the number 0 or 1, $R^5$ signifies hydrogen or lower alkyl and X signifies a 2-pyridyl residue optionally monosubstituted by lower alkyl, a 2-imidazolyl residue, a 2-imidazolinyl residue, a 2-thiazolyl residue, a 2-thiazolinyl residue or a 4(5)-imidazolyl residue optionally monosubstituted by lower alkyl,

and their acid addition salts.

2. Compounds in accordance with claim 1, wherein X signifies a 2-pyridyl residue optionally monosubstituted by lower alkyl, a 2-imidazolyl residue, a 2-imidazolinyl residue, a 2-thiazolyl residue or a 2-thiazolinyl residue.

3. Compounds in accordance with claim 2, wherein X signifies a 2-pyridyl residue optionally monosubstituted by lower alkyl.

4. Compounds in accordance with claim 3, wherein X signifies a methyl-substituted 2-pyridyl residue.

5. Compounds in accordance with claim 4, wherein the methyl group is in the 5- or 6-position.

6. Compounds in accordance with one of claims 1 to 5, wherein $R^5$ signifies hydrogen.

7. Compounds in accordance with one of claims 1 to 6, wherein n equals 0.

8. Compounds in accordance with one of claims 1 to 7, wherein $R^1$ together with $R^2$ and $R^3$ together with $R^4$ each signify an additional carbon-carbon bond.

9. 2-/[(5-Methyl-2-pyridyl)methyl]thio/-1H-naphth[2,3-d]imidazole and its acid addition salts.

10. 2-[(2-Pyridylmethyl)thio]-1H-naphth[2,3-d]imidazole and its acid addition salts.

11. Process for the manufacture of imidazole derivatives of the general formula I defined in Claim 1 and their acid addition salts, characterised by

a) for the manufacture of compounds of formula I, wherein n signifies the number 0, reacting a compound of the general formula

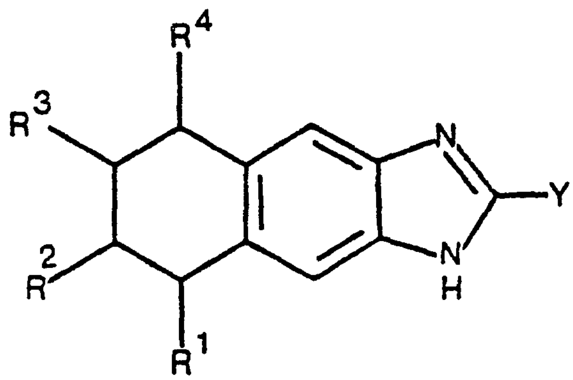

II

wherein $R^1$, $R^2$, $R^3$ and $R^4$ have the significance given above and Y has the significance given below,

with a compound of the general formula

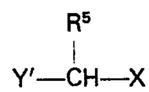

III

wherein $R^5$ and X have the significance given above and Y′ has the significance given below, whereby one of Y and Y′ signifies a mercapto group and the other signifies a leaving group, or

b) for the manufacture of a compound of formula I, wherein n signifies the number 1, oxidising a corresponding compound of formula I, wherein n signifies the number 0,

if desired, separating a diastereoisomeric mixture which may be obtained into the diastereoisomeric racemates and/or resolving a racemate which may be obtained into the two antipodes and/or, if desired, converting a free base obtained into an acid addition salt and/or converting an acid addition salt obtained into the free base or into another acid addition salt.

12. Process according to claim 11, characterised in that 2-/[(5-methyl-2-pyridyl)methyl]thio/-1H-naphth[2,3-d]imidazole or an acid addition salt thereof is manufactured.

13. Process according to claim 11, characterised in that 2-[(2-pyridylmethyl)thio]-1H-naphth-[2,3-d]imidazole or an acid addition salt thereof is manufactured.

14. Pharmaceutical preparations, containing an imidazole derivative of general formula I defined in claim 1 or an acid addition salt thereof.